# NEW EUROPEAN PATENT SPECIFICATION

(11) **EP 0 558 889 B2**
(45) Date of publication and mention of the opposition decision: **22.12.1999**
(45) Mention of the grant of the patent: 27.03.1996
(21) Application number: 93100289.3
(22) Date of filing: 29.05.1990
(51) Int. Cl.: A61F 13/52, A61L 15/60

(54) **Absorbent article comprising at least two superabsorbents**
Absorptionsartikel enthaltend wenigstens zwei Superabsorbern
Article absorbant comprenant au moins deux superabsorbants

(30) Priority: 31.05.1989 SE 8901964
(43) Date of publication of application: 08.09.1993
(62) Divisional of application: 90850217.2
(73) Proprietor: SCA Hygiene Products AB, 405 03 Göteborg (SE)
(72) Inventor: Roos, Anders, S-439 00 Onsala (SE); Quist, Magnus, S-448 00 Floda (SE); Hermansson, Jonas, S-510 20 Fritsla (SE)
(74) Representative: Hyltner, Jan-Olof

(56) References cited:
- EP-A- 0 343 941
- GB-A- 2 145 661
- US-A- 4 212 302
- US-A- 4 333 464
- US-A- 4 338 371
- US-A- 4 578 068
- US-A- 4 834 735

## Description

The present invention relates to an absorbent article, according to the preamble of claim 1.

Absorbent bodies or pads for disposable articles of the aforesaid kind, i.e. articles intended for one time use only, have, for many years, normally been produced essentially from absorbent fibre material. The fibre material used has most often been cellulose fluff material. In recent years, absorbent materials having a much greater absorption capacity than fluff have been developed and it has become progressively more general to incorporate these highly absorbent materials, so-called superabsorbents, in sanitary articles. Since the superabsorbents are able to absorb liquid in quantities many times greater than their own weight, the use of such materials in sanitary articles enables conciderable savings to be made in other kinds of absorption material, such as cellulose fluff. This is particularly desirable, since the articles can thereby be made thinner and therewith worn more discretely, which is a particularly important advantage in the case of adult incontinence.

It has been found, however, that although superabsorbents are advantageous with regard to their large liquid-absorbing capacities, they are also encumbered with certain drawbacks. For instance, when a superabsorbent absorbs liquid the phenomenom known as gel-blocking occurs. As a result of swelling, entrance to the non-utilized absorption capacity of the superabsorbent, which comprises a polymergel, is denied to the liquid and consequently liquid which is not absorbed, due to gel-blocking, will run in an uncontrolled fashion along the surface of the absorbent pad and ultimately leak from the pad and cause discomfort to the wearer. It is also found that different superabsorbents will absorb liquid at different rates. The absorption rate of some superabsorbents is too slow for the absorbents to have a practical use in sanitary products. Other superabsorbents have the drawback known as re-wetting, i.e. the gel is unable to retain the liquid contained therein when subjected to pressure and, consequently, liquid flows back to the surface material of the article, causing the surface material to become moist or wet and therewith subjecting the wearer to a great deal of discomfort.

Although several endeavours have been made to solve this problem, none of these endeavours have hitherto provided satisfactory results. A proposed solution to gel-blocking is disclosed, inter alia, in GB 2 145 661, according to which superabsorbent material is disposed in discrete layers with liquid-dispersing layers between the superabsorbent layers. The superabsorbents mentioned in this publication, however, are of mutually the same type and consequently the wearer is at the mercy of the properties of the superabsorbents with regard to the absorption properties of the sanitary article and its function. US 4,578,068 describes an absorbent pad which comprises a plurality of layers of fibre material with superabsorbents disposed between the layers. According to this publication, the superabsorbents may also be of mutually the same kind or may comprise a mixture of mutually different superabsorbents, although the properties of these superabsorbents is not disclosed.

The object of the present invention is to provide an absorbent article which comprises an absorbent pad having improved absorption properties with respect to earlier known absorbent pads, said absorbent pad combining the essential properties of an absorbent pad such as absorption rate and extremely good liquid-retention properties.

An absorbent article in accordance with the invention has the features stated in Claim 1.

It has surprisingly been found that the ability of the absorbent pad to absorb liquid can be substantially improved by incorporating in the pad two superabsorbents which differ from one another with respect to absorption rate and to re-wetting tendency respectively. It is highly important to the invention that the different superabsorbents are disposed in separate layers or in separate regions of the absorbent pad or body. It is found that the absorbent pad will have far better absorption properties when the superabsorbents are held separated from one another than if said absorbents are mixed together. Similarly, the incorporation of two superabsorbents will provide considerably better absorption than when only one of the superabsorbents is incorporated in the absorbent pad and the other excluded therefrom. The mutually relative location of the superabsorbents in the absorbent pad is also of great significance.

The absorbent article described in more detail with reference to an exemplifying embodiment illustrated in the accompanying drawings, is not an embodiment of the invention, but an example which is useful for the understanding of the invention.
Figure 1 is a view from above of an absorbent article with the side of the article which faces towards the wearer in use facing towards the viewer.
Figure 2 is a cross-sectional view of the article shown in Figure 1, taken on the line II-II in said Figure.
Figure 3 is a view from above of another embodiment of an absorbent article, with the side which faces the wearer in use facing towards the viewer.
Figure 4 is a cross-sectional view of the absorbent article shown in Figure 3, taken on the line IV-IV in said Figure.

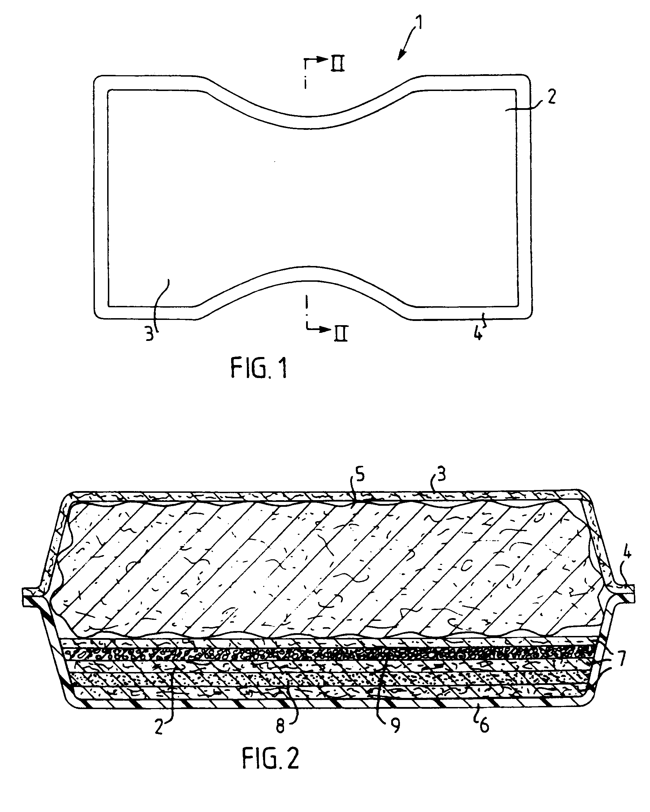

Figures 1 and 2 illustrate an absorbent article 1, which is assumed to be a disposable diaper and which comprises a liquid-permeable sheet 3, preferably made of nonwoven fibre fabric. The liquid-permeable sheet 3 is joined at the edge-parts 4 of the diaper to a liquid-impermeable sheet 6 in a conventional manner, e.g. with the aid of an adhesive binder. The liquid-impermeable sheet 6, which comprises polyethylene for instance, functions to prevent liquid from coming into contact with the wearer's clothes or to prevent liquid absorbed in the diaper from leaking therefrom.

Located beneath the liquid-permeable sheet 3 is a body of fibre wadding 5. The fibre wadding has a loose structure, is elastically resilient, and is shape-stable in both a dry and a wet state. The wadding comprises natural or synthetic fibres, such as polyester, polypropylene or polyethylene fibres, or mixtures thereof. So-called bicomponent fibres are suitable materials in this respect, by which is meant fibres composed of two types of polymer, for instance polypropylene/polyethylene, polyester/polyester or polyester/polyethylene. The fibre wadding 5 may also either be bound or non-bound with regard to its structure. Heat and adhesive bonding agents are examples of the means by which a fibre wadding structure can be bound. The adhesive binding agent may be a latex binder, for instance.

An absorbent body, generally referenced 2, is placed between the fibre wadding 5 and the liquid-impermeable sheet 6 distal from the wearer. The absorbent pad 2 comprises a plurality of distance-maintaining dispersion layers 7 and superabsorbent materials 8, 9 disposed between said layers. The absorbent pad of the Figure 2 embodiment includes three distance-maintaining dispersion layers 7, although this number may be greater than three. The layers 7 may, for instance, comprise one or more layers of tissue, although other materials, such as nonwoven or compressed cellulose fluff, may be used. It is important to the invention that the superabsorbent layers 8, 9 are held mutually separated and that the layer 9 is positioned closer to the wearer of the article than the layer 8. Thus, in the case of the Figure 2 embodiment, a tissue layer 7 is placed immediately beneath the wadding 5 and is followed sequentially by a superabsorbent layer 9, a tissue layer 7, a superabsorbent layer 8 and a tissue layer 7. The superabsorbents in the layers 8, 9 are preferably in powder form, although other forms are conceivable, such as a fibre form for instance. The superabsorbents in the layer 9 comprise a superabsorbent which exhibits very good liquid-retention properties, high surface-dryness and low re-wetting tendency. Such a superabsorbent is also characterized by a high gel strength. The superabsorbents in the layer 8, on the other hand, comprise a superabsorbent which exhibits a very high absorption rate.

The use of two superabsorbents having such radically different absorption properties as described above gives a highly surprising an unexpected result with regard to the absorption rate and re-wetting tendency of the absorbent pad as a whole. This will become more evident from the following example.

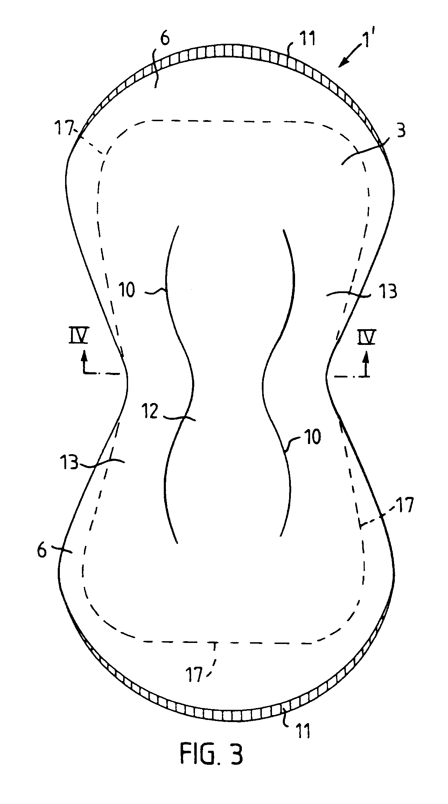

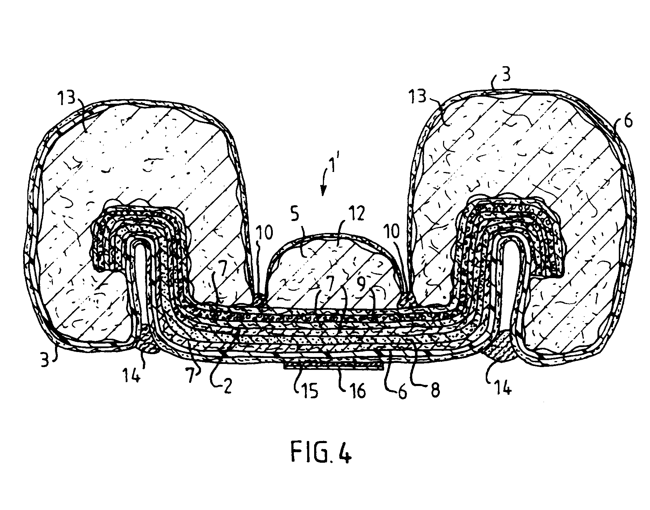

The absorbent article 1' illustrated in Figures 3 and 4 is assumed to be an incontinence guard. With regard to the choice of materials and positioning of the fibre wadding 5, absorbent pad 2 and liquid-impermeable sheet 6, the construction of the incontinence guard 1' is identical to that of the diaper 1. Like reference signs in Figures 1, 2 and 3, 4 identify identical elements.

The incontinence guard 1' differs from the diaper 1 in a number of respects, however.

Similar to the diaper 1 of Figures 1 and 2, the incontinence guard 1' comprises a liquid-permeable sheet 3, e.g. of nonwoven material, which is located nearest the wearer in use. This sheet, however, surrounds the incontinence guard around the whole of its periphery.

The incontinence guard 1' is divided by two longitudinally extending, curved fold lines 10, e.g. formed by weld seams or by adhesive bonds, into a central part 12 and two edge-parts 13. The intention of the fold lines 10 is to fold-up the edge-parts 13 along said lines and to fixate said upwardly folded edge-parts in a permanent upstanding position with the aid of joining means 14, e.g. a binding agent or a welded seam, such as to obtain a three-dimensional basin-like absorbent article in which the edge-parts 13 function as sealing embankments in the lateral direction of the article.

The parts 12, 13 are relatively elongated, i.e. their extension in the longitudinal direction of the article is greater than the extension of the article in its transverse direction. The end-parts of the incontinence guard are provided with joins 11, which may, for instance, have the form of welded joins or adhesive joins.

When the fold lines 10 comprise welded joins or seams, such joins can be produced by appropriate, conventional welding techniques, e.g. ultrasonic welding, impulse welding or high frequency welding. The fold lines 10 may also have a linear or arcuate shape, as an alternative to the curve shape shown in Figure 3.

The fold lines 10 may either be continuous or discontinuous. For instance, the fold lines may be formed by a broken curve of punctiform welds.

Located immediately beneath the absorbent pad 2, as seen from the wearer, is a liquid-impermeable sheet 6, which extends slightly up along the edge-parts and end-parts of the incontinence guard 1' and somewhat in over the fibre wadding 5 on the side surface of the incontinence guard 1' facing towards the wearer in use. The extension of the liquid-impermeable layer 6 is shown with a broken line 17 in Figure 3.

Positioned on the underside of the incontinence guard is an adhesive layer 15, by means of which the guard can be removably attached to the underclothes of the wearer. Prior to using the guard 1', the adhesive layer 15 is protected by a protective layer 16, which is preferably siliconized or treated with a release agent in some other way, so that when the guard 1' is used, the protective material can be readily removed and therewith render the adhesive layer 15 active.

The absorption properties of an absorbent article with respect to re-wetting tendencies will now be illustrated in the following example.

### Example 1

The absorption rate of six superabsorbents was measured in accordance with the Vortex-analysis method. The six superabsorbents tested were all available commercially and sold under the trade names Aqualic CA W-4, Waterlock J 550, Aridall 1125, Aridall 1092, Sanwet IM 2200 D and Sanyo PA 200G.

A beaker containing 50 ml NaCl (aq) in a quantity of 9 g/liter was placed on a magnetic stirrer. The stirrer was set to rotate at a speed of 600 ± 20 rpm. When the resultant Vortex has stabilized, 2.0 g of superabsorbent were poured into the beaker, externally of the Vortex. A measurement was taken of the time that lapsed from the moment of adding the superabsorbent to the beaker to the moment that the Vortex disappeared and a completely smooth surface was obtained. The procedure was repeated the requisite number of times.

The following results were obtained:

**Table 1**

| Superabsorbent | Vortex time (s) |
|---|---|
| Aqualic CA W-4 | 21 |
| Aridall 1092 | 5 |
| Aridall 1125 | 7 |
| Sanwet IM 2200 D | 67 |
| Sanyo PA 200 G | 44 |
| Waterlock J 550 | 2 |

Waterlock J 550, Aridall 1092 and Aridall 1125 were found to have much shorter absorption times than the other superabsorbents tested. Waterlock J 550 is used in Examples 3-5 as an example of a superabsorbent which possesses a very high absorption rate.

### Example 2

Measurements were made of the gel strength of the same superabsorbents as those whose absorption rates were measured in Example 1.

The gel strength measurements were taken with the aid of a Carri-Med C.S. rheometer. The measurements were taken with the following parameters:

| | |
|---|---|
| Measuring program | Oscillation |
| Measuring system | Plate-plate (serrated) |
| Plate radius | 2 cm |
| Plate distance | 2 mm |
| Oscillation frequency | 1.0 Hz |
| Torque | 1.00 Nm |
| Temperature | 20°C |

30 ml of 0.9% NaCl-solution were poured into a 100 ml beaker. 1.00 g of superabsorbent was added to the solution, while stirring with a magnetic stirrer. Stirring was stopped when the superabsorbent had absorbed the total quantity of liquid present, whereafter the resultant gel was allowed to stand for one hour. The gel was then mixed and a sample of 5 ml was taken. The sample was placed centrally on the bottom plate, whereafter the measuring process was commenced.

Table 2 shows the values obtained with an energy input of 3 mJ.

**Table 2**

| Superabsorbent | Gel strength (kPa) |
|---|---|
| Aqualic CA W-4 | 3.2 |
| Aridall 1092 | 0.2 |
| Aridall 1125 | 0.3 |
| Sanwet IM 2200 D | 2.9 |
| Sanyo PA 200 G | 2.9 |
| Waterlock J 550 | 1.4 |

Aqualic CA W-4, Sanwet IM 2200 D and Sanyo PA 200 G exhibited high gel strength, whereas the remaining superabsorbents exhibited moderate or low gel strength.

A relationship between high gel strength and low re-wetting tendency, i.e. high liquid-retention, can be shown even though no clear relationship exists.

Without intending to provide a complete explanation of this relationship, it can be mentioned that a strongly cross-linked, dense and hard gel is more difficult to compress than a gel having the opposite properties, and consequently it is more difficult to press absorbed liquid from a gel of the firstmentioned kind than from the lastmentioned kind. Furthermore, the greater suction force of a strongly cross-linked superabsorbent contributes to the low re-wetting tendency of said absorbent. Higher degrees of cross-linking result in finer capillary systems, which in turn result in higher suction forces. Low re-wetting tendencies can also be obtained by subjecting the gel to different types of surface treatment.

### Example 3

The superabsorbents recited in Examples 1 and 2 were tested for re-wetting.

5 g of a superabsorbent were spread uniformly in the form of a layer in the bottom, rectangular absorbent pad of a diaper sold under the trade name Libero Super. 100 ml of an 0.9% NaCl-solution were poured through a pipe onto the wetting-location of the diaper. The time taken for the diaper to absorb the liquid was measured and recorded. After 10 minutes, filter papers, having a diameter of 80 mm, were placed over the wetting-location and subjected to a load of 1.5 kg for 15 seconds. The filter papers were weighed before and after applying said load and re-wetting was recorded.

The method provides a measurement of the ability of the superabsorbent to absorb liquid and to retain said liquid when subjected to load, when the superabsorbent is incorporated in an absorbent article.

The results are shown in Table 3.

**Table 3**

| Superabsorbent | Re-wetting (ml) |
|---|---|
| Aqualic CA W-4 | 1 |
| Aridall 1092 | 21 |
| Aridall 1125 | 10 |
| Sanwet IM 2200 D | 6 |
| Sanyo PA 200 G | 1 |
| Waterlock J 550 | 12 |

Aqualic CA W-4 and Sanyo PA 200 G were found to have by far the lowest re-wetting tendency of the superabsorbents tested, this being in agreement with the gel strength measurements of Example 2. Aqualic CA W-4 was used in the following examples as an example of a surface-dry superabsorbent having very high liquid-retention properties.

### Example 4

Absorption rate, liquid leakage in sloping planes and re-wetting were measured in respect of an absorbent pad or body which incorporated two mutually separate superabsorbent layers. The results are compared with the results obtained in respect of a reference body which included an absorbent pad comprising a layer of a mixture of the superabsorbents.

The firstmentioned absorbent pad comprised an upper tissue-laminate in which 2.5 g Aqualic CA W-4 were placed in powder form, and a bottom tissue-laminate which contained 2.5 g Waterlock J 550 relative to the liquid-receiving surface material.

The last mentioned absorbent body included a tissue laminate which contained a mixture of varying quantities Aqualic CA W-4 and Waterlock J 550.

All reference bodies included a nonwoven material , manufactured by Shiseida, and two pieces of fibre wadding, sold under the trade mark Dacron, placed on top of the absorbent pad.

### Absorption rate

The reference bodies were laid flat and 100 ml 0.9 % NaCl (aq) were poured onto respective bodies over a period of 7 seconds and the time taken for the body to absorb the liquid was measured (visual observation). The results are presented in Table 4 below.

### Liquid leakage in sloping planes

The reference body was placed on an inclined surface, the angle of said surface to the horizontal being 30%. 100 ml of 0.9% NacL (aq) were poured onto the upper end of the body over a period of 7 seconds. The quantity of non-absorbed liquid was collected at the lower end of the body and weighed. The results are presented in Table 4 below.

### Re-wetting

A plate weighing 3.5 kg was laid on top of the reference body. 50 ml of 0.9% Nacl (aq) were poured onto the reference body and the body was left to absorb the liquid for a period of 3 minutes.

Filter papers weighing 5 g and having a diameter of 50 mm were placed on the wetting location and loaded with a pressure of 8 kg/dm² for 1 minute.

The filter papers were then weighed and the increase in weight of said papers taken as a re-wetting measurements. The results are presented in Table 4 below.

Aqualic CA W-4 is referenced AL and Waterlock J 550 is referenced WL.

**Table 4**

| Type of abs.body | Quantity | | Absorption-time (s) | Leakage in sloping plane (g) | Re-wetting (g) |
|---|---|---|---|---|---|
| | AL (g) | WL (g) | | | |
| Mixing | 4 | 1 | 5.9 | 11.0 | 3.2 |
| Mixing | 3 | 2 | 3.0 | 9.5 | 3.1 |
| Mixing | 2 | 3 | 2.2 | 9.7 | 3.6 |
| Mixing | 1 | 4 | 1.6 | 8.7 | 3.5 |
| Separate layers | 2.5 | 2.5 | 2.1 | 0 | 0.6 |

The reference bodies which comprised a relatively large proportion of rapidly-absorbing superabsorbent, in the example at least 2/5, were found to have a short absorption time, irrespective of whether the superabsorbents were mixed or separated, although the reference body in which the superabsorbents were mutually separated was found to have considerably better values with regard to liquid leakage in a sloping plane and re-wetting than the reference bodies which included a layer comprising a mixture of the same superabsorbents.

### Example 5

Absorption rate, liquid leakage in a sloping plane and re-wetting were measured in respect of a series of reference bodies which contained various different superabsorbents placed in mutually separate layers.

The analyses were carried out in the same manner as that recited in Example 4. In all cases, the absorbent pad comprised an upper tissue-laminate containing 2.5 g superabsorbent, and a lower tissue-laminate containing 2.5 g of a superabsorbent different to the superabsorbent in the upper laminate.

The values obtained are presented in Table 5 below.

**Table 5**

| Absorbent pad | | Absorption time (s) | Leakage in sloping plane (g) | Re-wetting (g) |
|---|---|---|---|---|
| U-layer | L-layer | | | |
| AL | S | 9.6 | 14.6 | 1.1 |
| AL | A10 | 4.8 | 15.0 | 2.5 |
| AL | A11 | 7.2 | 15.8 | 1.7 |
| AL | SW | 13.8 | 21.0 | 1.0 |
| AL | WL | 2.1 | 0 | 0.6 |
| WL | S | 2.6 | 11.7 | 2.7 |
| WL | A10 | 2.3 | 8.4 | 3.4 |
| WL | A11 | 2.9 | 12.4 | 4.1 |
| WL | SW | 2.7 | 10.7 | 3.1 |
| WL | AL | 2.0 | 7.3 | 2.0 |
| U-layer = Upper layer L-layer = Lower layer AL = Aqualic CA W-4 WL = Waterlock J 550 S = Sanyo PA 200 G A 10 = Aridall 1092 A 11 = Aridall 1125 SW = Sanwet IM 2200 D | | | | |

The results show that the absorption time is much shorter in those instances when the superabsorbent which in Example 1 exhibited the fastest absorption rate, Waterlock J 550, is present. The lowest re-wetting tendency is obtained with those absorbent pads which contain Aqualic CA W-4, which in Example 2 was found to have the highest gel strength and, in Example 3, a very low re-wetting tendency.

The combination of a superabsorbent of low re-wetting tendency in an upper layer and a superabsorbent of high absorption rate in a lower layer gives by far the best result with respect to liquid-leakage in a sloping plane. The re-wetting tendency is also lowest with this combination.

### Example 6

Absorption rate, liquid-leakage in a sloping plane and re-wetting were measured in respect of a series of reference bodies in which the superabsorbents were incorporated in respective reference bodies in varying quantities, said measurements being taken in the same manner as that recited in Examples 4 and 5. The total amount of superabsorbent in each body was 5 g. The absorbent pads comprised an upper tissue-laminate of Aqualic CA W-4, and a lower tissue-laminate of Waterlock J 550. In some instances, one of the superabsorbents was omitted, i.e. the absorbent pad in question comprised solely one single-laminate containing one single superabsorbent.

The average measurement values are presented in Table 6 below. Aqualic CA W-4 is abbreviated to AL in Table 6 and Waterlock J 550 is abbreviated to WL.

**Table 6**

| Amount of superabsorbent (g) | | Absorption time (s) | Leakage in sloping plane (g) | Re-wetting (g) |
|---|---|---|---|---|
| AL | WL | | | |
| 5 | 0 | 13.98 | 6.0 | 0.88 |
| 4 | 1 | 3.09 | 2.1 | 0.58 |
| 3 | 2 | 2.29 | 0 | 0.47 |
| 2.5 | 2.5 | 2.14 | 0 | 0.46 |
| 2 | 3 | 2.80 | 0.5 | 0.64 |
| 1 | 4 | 1.25 | 0 | 0.81 |
| 0 | 5 | 0.97 | 2.3 | 3.36 |

It will be seen from Table 6 that the absorption time is much longer in those cases when the rapidly-absorbing superabsorbent is not present than in other cases. A higher re-wetting value is obtained for the absorbent pad which contains solely Waterlock J 550 than for the other absorbent pads. The values obtained in respect of liquid-leakage in a sloping plane show that the presence of both types of superabsorbent provides the best results.

In accordance with the invention, instead of placing the superabsorbents in vertically superimposed relationship, the superabsorbent which exhibits a low re-wetting tendency can be placed nearest the liquid-receiving region (the so-called wetting location) and the rapidly-absorbing superabsorbent can be placed in a separate region outside said wetting location, for instance in a circular region around said wetting location.

Furthermore, as will be understood, an absorbent pad can be constructed from more than two mutually different superabsorbents having substantially differing absorption properties.

The superabsorbents are placed between at least two layers of material, i.e. in a laminate form.

The aforegoing exemplifying examples with reference to a diaper or an incontinence guard can also be applied to other absorbent articles, such as sanitary towels or panty-protectors for example.

## Claims

1. An absorbent article intended for one-time-use only, such as a diaper, incontinence guard, a sanitary towel or the like, comprising a liquid-permeable sheet (3), which faces the wearer in use, an absorbent pad (2), and a liquid-impermeable sheet (6) which is distal from the wearer in use, characterized in that the absorbent pad includes at least two mutually different superabsorbents being disposed between at least two material layers in separate regions of the absorbent pad, a superabsorbent which has a high liquid-retention ability when subjected to pressure, located nearest the liquid-receiving region, the so called wetting location, of the absorbent pad and another superabsorbent which exhibits a high absorption rate located outside said wetting location.

2. An absorbent article according to claim 1, characterized in that the superabsorbent which exhibits a high absorption rate is placed in a region around said wetting location in which the superabsorbent having a high liquid-retention ability is placed.

## Patentansprüche

1. Absorbierender Artikel zur Einmalverwendung, beispielsweise eine Windel, ein Inkontinenzschutz, eine Damenbinde oder dergleichen, mit einer flüssigkeitsdurchlässigen Lage (3), die beim Gebrauch auf die Trägerperson zuweist, einem absorbierenden Kissen (2) und einer flüssigkeitsundurchlässigen Lage (6), die beim Gebrauch von der Trägerperson entfernt ist, **dadurch gekennzeichnet, daß** das absorbierende Kissen wenigstens zwei zueinander verschiedene Superabsorbens, die zwischen wenigstens zwei Materialschichten in separaten Bereichen des absorbierenden Kissens angeordnet sind, enthält, nämlich ein Superabsorbens, das, wenn es einem Druck ausgesetzt wird, eine hohe Flüssigkeits-Rückhaltefähigkeit hat und das dem flüssigkeitsaufnehmenden Bereich, der sogenannten Nässungsstelle, des absorbierenden Kissens am nächsten liegt, sowie ein weiteres Superabsorbens, das eine hohe Absorptionsgeschwindigkeit aufweist und außerhalb der Nässungsstelle liegt.

2. Absorbierender Artikel nach Anspruch 1, **dadurch gekennzeichnet, daß** das eine hohe Absorptionsgeschwindigkeit aufweisende Superabsorbens in einem Bereich um die Nässungsstelle herum angeordnet ist, in der das Superabsorbens liegt, das eine hohe Flüssigkeits-Rückhaltefähigkeit aufweist.

## Revendications

1. Article absorbant à usage unique, comme une couche-culotte, une protection pour incontinents, une serviette hygiénique ou analogue, comprenant une feuille (3) perméable aux liquides, qui fait face à l'utilisateur quand l'article est porté, un coussin absorbant (2) et une feuille (6) imperméable aux liquides qui se trouve loin de l'utilisateur quand l'article est porté,
caractérisé en ce que le coussin absorbant contient au moins deux superabsorbants différents l'un de l'autre disposés entre au moins deux couches de matière dans des régions distinctes du coussin absorbant, à savoir un superabsorbant ayant une grande aptitude à la rétention du liquide quand il est soumis à une pression, situé le plus près de la région recevant le liquide, dite zone de mouillure, du coussin absorbant et un autre superabsorbant présentant une grande vitesse d'absorption, situé à l'extérieur de ladite zone de mouillure.

2. Article absorbant selon la revendication 1, caractérisé en ce que le superabsorbant qui présente une grande vitesse d'absorption est situé dans une région entourant la zone de mouillure dans laquelle est placé le superabsorbant ayant une grande aptitude à la rétention du liquide.
